# EUROPÄISCHE PATENTSCHRIFT

(11) **EP 3 478 412 B1**
(45) Veröffentlichungstag und Bekanntmachung des Hinweises auf die Patenterteilung: **15.04.2020**
(21) Anmeldenummer: 17740308.6
(22) Anmeldetag: 29.06.2017
(51) Int. Cl.: B01L 3/00, G01N 1/40, A61B 5/15, B65D 83/04

(54) **VORRICHTUNG ZUM BEREITSTELLEN VON EINE GETROCKNETE FLÜSSIGKEITSMENGE, INSBESONDERE BLUT, AUFWEISENDEN, SAUGFÄHIGEN PROBENTRÄGERN**
DEVICE FOR MAKING AVAILABLE ABSORBENT SAMPLE CARRIERS HAVING A QUANTITY OF DRIED LIQUID, IN PARTICULAR BLOOD
DISPOSITIF DE PRÉPARATION DE PORTE-ÉCHANTILLONS ABSORBANTS COMPRENANT UNE QUANTITÉ DE LIQUIDE SÉCHÉE, EN PARTICULIER DU SANG

(30) Priorität: 30.06.2016 DE 102016211911
(43) Veröffentlichungstag der Anmeldung: 08.05.2019
(73) Patentinhaber: Sarstedt AG & Co. KG, 51588 Nürnbrecht (DE)
(72) Erfinder: WEINSTOCK, Mark, 57612 Helmenzen (DE)
(74) Vertreter: Grosse, Wolf-Dietrich Rüdiger
(86) Internationale Anmeldenummer: PCT/EP2017/066177
(87) Internationale Veröffentlichungsnummer: WO 2018/002249

(56) Entgegenhaltungen:
- WO-A1-2013/067520
- DE-A1-102013 201 505
- US-A- 4 174 048
- US-A1- 2009 314 795
- US-A1- 2013 292 398
- US-B1- 6 523 717

## Beschreibung

Die Erfindung betrifft eine Vorrichtung zum Bereitstellen von eine getrocknete Flüssigkeitsmenge, insbesondere Blut, aufweisenden, saugfähigen Probenträgern zu analytischen Auswertungen, wobei eine aufgegebene unbestimmte Flüssigkeitsmenge durch Kapillarwirkung auf saugfähige Probenträger appliziert wird und die Probenträger nach dem Trocknen der Flüssigkeitsmenge an eine weiterverarbeitende Einheit, beispielsweise eine Testplatte, abgegeben werden.

Die sogenannte Dried-Blood-Spot (DBS)-Analyse ist seit langem für verschiedenartige klinische Untersuchungen an Blutproben, aber auch auf anderen Anwendungsfeldern, wie in der Pharmaforschung, der klinischen Chemie, der therapeutischen Arzneimittelüberwachung oder der forensischen Toxikologie und der Dopinganalytik bekannt, vor allem in Verbindung mit nachweisstarken LC-MS/MS (liquid chromatography tandem mass spectroscopy)-Systemen. Zur DBS-Analyse reichen wenige aus der Fingerbeere oder Ferse eines Patienten entnommene Blutstropfen aus. Das entnommene Blut wird, wie beispielsweise aus der US 8 586 382 B2 bekannt, auf speziellem, saugfähigem Testpapier als Probenträger von Hand in kreisförmig markierte Bereiche aufgetragen und nach seiner Trocknung und ggf. vorheriger Aufbewahrung zu einer Analyse gegeben, was in einer weiterverarbeitenden Einheit in einen automatisierten Prozessablauf eingebunden werden kann. Da es sich bei dem aufgetragenen, getrockneten Blut um eine unbestimmte Flüssigkeitsmenge handelt, ist es auf jeden Fall vorab erforderlich, aus dem Probenträger bzw. den markierten Bereichen des Testpapiers eine Scheibe von wenigen Millimetern Durchmesser des darauf getrockneten und verteilten Gutes für die nachfolgende Untersuchung zu desorbieren, um für die Analyse bzw. Diagnostik eine definiertere Blutmenge bereitstellen zu können. Die Scheibe wird mit einem geeigneten Lösemittel gespült. Nach einer Aufbereitung des so gewonnenen Extraktes zur Separierung des Lösungsmittels steht das Gut für eine Analyse zur Verfügung.

Durch die WO 2015/044454 A2 ist es zur Verbesserung der Auswerte- bzw. Messergebnisse bekannt geworden, die aus einem Probenträgerpapier, insbesondere durch Ausstanzen, zu desorbierende Menge getrockneten Bluts durch Kapillarkanäle exakter zu bestimmen. Dazu wird das unbestimmt entnommene Flüssigkeitsvolumen in mehrere, jeweils eine bestimmte Blutmenge aufnehmende Kapillarkanäle eingeleitet, nach deren völliger Befüllung der Zufluss an der Eingabestelle abreißt. So gelangt in der Folge am Auslaufende eines jeden Kapillarkanals nur exakt die durch das Aufnahmevolumen des Kapillarkanals bestimmte bzw. kalibrierte Blutsmenge in einen zur Ausstanzung vorgesehenen Bereich mit einem Durchmesser von etwa 6 mm auf das Probenträgerpapier bzw. eine Probenträgersammelkarte. Damit die kalibrierte Blutsmenge auf die Probenträgersammelkarte übergeleitet werden kann, ist am Auslaufende der Kapillarkanäle eine lösliche, nach dem Auflösen den Durchfluss erlaubende Membrane ausgebildet.

Dosiervorrichtungen für verschiedenste Objekte sind beispielsweise aus US4174048, US6523717B1, US2009/0314795A1 und US2013/0292398A1 bekannt. Halterungen für absorbierende Probenträger sind beispielsweise aus WO2013/067520A1 bekannt.

Ausgehend von diesem Stand der Technik soll eine Vorrichtung geschaffen werden, mit der sich in einfacherer und für den Anwender gegenüber der Berührung mit der Flüssigkeit, insbesondere Blut, sichereren Weise, insbesondere ohne das Erfordernis einer Desorption einzelner Probenträger aus einer Probenträgersammelkarte oder dergleichen, Probenträger zur Analyse bereitstellen lassen. Die erfindungsgemäße Vorrichtung stellt insbesondere eine Alternative zum Stand der Technik dar.

Diese Aufgabe wird durch den Gegenstand des Anspruchs 1 gelöst. Dieser ist dadurch gekennzeichnet, dass das Haltemittel elastisch ausgebildet ist einerseits zum Halten der Probenträger in dem Aufnahmeraum des Röhrchens und andererseits zum kontrollierten vereinzelten Freigeben der Probenträger aus dem Aufnahmeraum unter Krafteinwirkung durch den Stößel, dass an dem Stößel zumindest im Bereich seines Stößelgriffs eine Mehrzahl von Flügeln in Längsrichtung des Stößels beabstandet zueinander angeordnet ist, wobei benachbarte der Flügel in Umfangsrichtung des Stößels jeweils um einen vorbestimmten Umfangswinkel zueinander versetzt sind, und dass an dem hinteren Ende des Röhrchens eine Durchgangsöffnung vorgesehen ist, welche ausgebildet ist, die Flügel des Stößels nur in mindestens einer vorbestimmten Umfangswinkellage, nachfolgend auch Durchlass-Winkellage genannt, passieren zu lassen.

Die beanspruchte Vorrichtung fungiert vorteilhafterweise als Dosier-Spender für jeweils genau einen Probenträger. Durch eine Krafteinwirkung auf das hintere Ende des Stößels, aufgebracht z. B. durch den Daumen einer Bedienperson, wird der Stößel nicht etwa beliebig weit, sondern nur um eine vorbestimmte diskrete Weglänge in das Innere des Röhrchens hinein verschoben. Diese Weglänge entspricht in etwa dem Abstand zwischen zwei benachbarten Flügeln. Diese Weglänge entspricht in etwa auch dem Durchmesser bzw. der Länge eines Probenträgers; deshalb wird sie nachfolgend auch als Auswurfhub bezeichnet. Die Begrenzung der Weglänge wird durch die beanspruchte Merkmalskombination sichergestellt. Konkret kann ein zunächst noch außerhalb des Röhrchens befindlicher voreilender Flügel die Durchgangsöffnung an dem hinteren Ende des Röhrchens nur dann passieren, wenn seine Umfangswinkellage mit der Durchlass-Winkellage der Durchgangsöffnung korrespondiert. In diesem Fall kann der Stößel dann aber nur um einen Auswurfhub in das Innere des Röhrchens hineingedrückt werden, bis der nächste benachbarte zunächst noch außerhalb des Röhrchens befindliche nacheilende Flügel auf die Durchgangsöffnung trifft. Diese Durchgangsöffnung mit ihrer Durchlass-Winkellage für den voreilenden Flügel ist dann für den nacheilenden Flügel aufgrund von dessen Versatz in Umfangsrichtung zunächst gesperrt. Die Durchgangsöffnung bildet insofern einen Anschlag für den nachfolgenden Flügel und dient insofern als Wegbegrenzung für den Stößel. Erst wenn der Stößel dann in Umfangsrichtung gedreht wird, so dass die Winkellage des nacheilenden Flügels mit der Durchlass-Winkellage der Durchgangsöffnung korrespondiert, kann der Stößel um eine weitere Weglänge, d.h. um einen weiteren Auswurfhub weiter in das Innere des Röhrchens hineingedrückt und damit der nächste Probenträger aus dem Röhrchen herausgedrückt werden.

Die Krafteinwirkung bzw. der Druck auf den Stößelgriff überträgt sich jeweils in Längsrichtung des Stößels und des Röhrchens über den Stößelkopf von dem hintersten auf den vordersten der in einer Reihe bzw. gestapelt in dem Aufnahmeraum des Röhrchens angeordneten Probenträger. Der vorderste Probenträger drückt dann unmittelbar auf das Haltemittel. Ist die Krafteinwirkung groß genug, so gibt das Haltemittel aufgrund seiner Elastizität nur den jeweils vordersten Probenträger schließlich frei, so dass dieser aus dem Röhrchen nach außerhalb entweichen kann.

Die beanspruchte Vorrichtung dient, wie gesagt, insbesondere dazu, Probenträger vereinzelt an eine Testplatte abzugeben. Bei einem solchen Abgabevorgang liegt die Testplatte typischerweise auf einem horizontalen Tisch auf und die erfindungsgemäße Vorrichtung bzw. das Röhrchen wird senkrecht dazu gehalten.

In der vorliegenden Beschreibung werden sämtliche Begriffe beispielhaft bezogen auf diese Anordnung beschrieben. Konkret meint deshalb der Begriff "vorderes Ende" das der Testplatte zugewandte Ende des Röhrchens. Es ist das Ende, wo das Haltemittel angeordnet ist und aus welchem der jeweils vorderste Probenträger ggf. freigegeben bzw. ausgeworfen wird. Dementsprechend meinen die gleichbedeutenden Begriffe "unterster Probenträger" oder "vorderster Probenträger" den an dem Haltemittel direkt anliegenden Probenträger, der als nächster ausgeworfen wird.

Umgekehrt meint der Begriff "hinteres Ende" des Röhrchens, das der Testplatte und dem Haltemittel abgewandte Ende des Röhrchens bzw. jenes dem Auswurfende gegenüberliegende Ende, wo der Stößel gelagert ist. Der Begriff "hinterster Probenträger" meint insofern analog den Probenträger an dem von dem Haltemittel entfernten Ende des Stapels von Probenträgern. Der Begriff "hinterster Probenträger" ist gleichbedeutend mit dem Begriff "oberster Probenträger".

Die Probenträger mit ihrer jeweils vorbestimmten Aufnahmekapazität für die Flüssigkeit sind selbst die Führungsgröße zur Bestimmung der jeweiligen, exakten Teilmengen aus der unbestimmt aufgegebenen Flüssigkeitsmenge. Hierdurch lässt sich vermeiden, dass das System schon vor der Trocknung der bestimmten Flüssigkeits- bzw. Blutmenge aufgelöst werden muss. Das durch einen Verschluss geschlossene System verhindert daher mit größter Sicherheit, dass flüssiges Blut nach außen dringen kann. Dieses wird vielmehr nur intern durch die Kapillarwirkung der saugfähigen, porösen Probenträger gezielt an die einzelnen Probenträger weitergeleitet, wobei jeder Probenträger jeweils nur ein vorher festgelegtes (Teil)-Volumen der Gesamtflüssigkeitsmenge aufnimmt. Erst wenn die Teilmenge getrocknet ist, wird das System geöffnet und die Probenträger werden sequentiell ausgeworfen, wie oben beschrieben. Ein Ausstanzvorgang, wie im Stand der Technik erforderlich, entfällt dabei völlig, denn die Probenträger liegen in dem bei aufgesteckter oder - geschraubter Kappe geschlossenen System schon vereinzelt vor.

Zur Aufnahme einer bestimmten Teilmenge der Flüssigkeit eignen sich Probenkörper, die aus verpressten Kunststoffkugeln bestehen, deren natürliche Oberflächenbeschaffenheit eine Polarität besitzt, die eine kapillarfördernde Wirkung hat. Optional lassen sich die Kunststoffkugeln durch eine Oberflächenbehandlung in einen saugfähigen Zustand versetzen. Das Aufnahmevolumen der Probenträger lässt sich dabei durch die Zwischenräume der einzelnen Kugeln bestimmen, so dass Variationen durch die Wahl der geometrischen Abmessungen möglich sind. Nach der aufeinanderfolgenden Füllung der saugfähigen Probenkörper mit der durch sie jeweils bestimmten Flüssigkeits-Teilmenge (Aufnahmekapazität) ist kein weiterer Kapillarzug mehr vorhanden. Ein Überschuss der aufgegebenen Flüssigkeitsmenge verbleibt in dem geschlossenen System, wozu der oberste, d.h. der zuerst eingebrachte Probenkörper der Stapellage als Puffer bzw. Reservoir dienen kann. Denn der Anwender erkennt bei dem aus einem transparenten, d. h. durchsichtigen Material, z.B. Glas oder Kunststoff, bestehenden Röhrchen sofort das Erreichen des Sättigungsgrades, und zwar sobald das Blut in den obersten Probenträger eintritt. Die Zufuhr der unbestimmten Blutsmenge kann daraufhin unverzüglich unterbrochen werden. Das Blut kann z. B. aus einer Pipette oder nach Entnahme aus der Fingerbeere eines Patienten oder beispielweise auch dem Ohrläppchen eines Tieres mittels einer Kanüle bereitgestellt werden. Das Röhrchen wird dann nur zur Entnahme der Probenkörper nach der Trocknung der Flüssigkeit durch Entfernen der Kappe geöffnet.

Weitere Merkmale und Einzelheiten der Erfindung sind Gegenstand der abhängigen Ansprüche.

Der Beschreibung sind fünf Figuren beigefügt. Es zeigen:
- Fig. 1: ein Probenträger-Röhrchen in einer perspektivischen Gesamtansicht im Auslieferungszustand;
- Fig. 2: eine perspektivische Gesamtansicht, wie zuvor in Fig. 1, demgegenüber mit zur Einfüllung einer unbestimmt aufgegebenen Blutsmenge entfernter Kappe, so dass die Probenträger zugänglich sind;
- Fig. 3: eine gegenüber Fig. 2 andere perspektivische Gesamtansicht, welche einen Einblick in das offene vordere Ende des Röhrchens gewährt;
- Fig. 4: das Röhrchen der Fig. 3 mit zur Trocknung der Probenträger und zum Transport aufgesetzter Verschlusskappe;
- Fig. 5: das Röhrchen der Fig. 3 in einer Ausgangsposition aufgesetzt auf eine Testplatte zum Auswerfen des untersten Probenträgers; und
- Fig. 6: das Röhrchen aufgesetzt auf die Testplatte gemäß Fig. 5 mit dem abgeworfenen vordersten Probenträger.

Die Erfindung wird nachfolgend unter Bezugnahme auf die genannten Figuren in Form von Ausführungsbeispielen detailliert beschrieben. In allen Figuren sind gleiche technische Elemente mit gleichen Bezugszeichen bezeichnet.

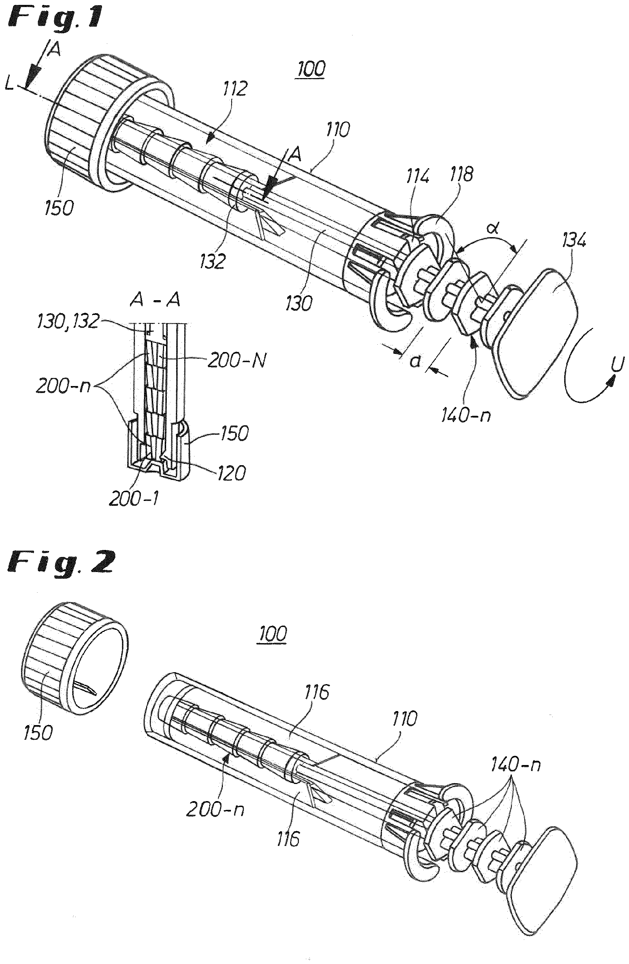

Figur 1 zeigt eine perspektivische Gesamtansicht der erfindungsgemäßen Vorrichtung 100 zum Bereitstellen von Probenträgern 200-n mit 1 < 1 < n < N mit n, N aus der Menge der natürlichen Zahlen. Jeder der Probenträger 200-n ist ausgebildet, eine vorbestimmte Menge einer Flüssigkeit, beispielsweise Blut aufzunehmen.

Die erfindungsgemäße Vorrichtung 100 umfasst ein Röhrchen 110 mit einem Aufnahmeraum 112 zur Aufnahme einer Mehrzahl der Probenträger 200-n. Der Aufnahmeraum 112 im Inneren des Röhrchens 110 ist so gestaltet, dass in ihm die einzelnen Probeträger 200-n in Längsrichtung L des Röhrchens 110 gestapelt angeordnet werden können. An seinem vorderen Ende ist das Röhrchen 110 mit einem Verschluss 150, beispielsweise in Form einer Kappe oder eines Stopfens verschlossen. An seinem hinteren, dem vorderen Ende gegenüberliegenden Ende ist in Längsrichtung des Röhrchens ein Stößel 130 verschiebbar gelagert. Dieser Stößel 130 taucht mit seinem einen Ende in Form eines Stößelkopfes 132 in das Röhrchen 110 hinein zum Begrenzen des Aufnahmeraumes 112 für die Probenträger. Mit seinem anderen gegenüberliegenden Ende in Form eines Stößelgriffes 134 ragt der Stößel aus dem Röhrchen 110 heraus.

An dem Stößel ist zumindest in dem Bereich seines Stößelgriffes 134 eine Mehrzahl von Flügeln 140-n angeordnet. Die Flügel sind jeweils in Längsrichtung L des Stößels um einen vorbestimmten Abstand a beabstandet zueinander angeordnet. Außerdem sind jeweils zwei benachbarte Flügel in Umfangsrichtung U des Stößels 130 um einen vorbestimmten Umfangswinkel α, mit beispielsweise α = 90°, zueinander versetzt.

Das dem vorderen Ende gegenüberliegende hintere Ende des Röhrchens 110 weist eine Durchgangsöffnung 114 auf, welche ausgebildet ist, die Flügel 140-n des Stößels 130 nur in einer vorbestimmten Umfangswinkellage passieren zu lassen.

Die Flügel 140-n erstrecken sich jeweils radial von dem Stößel 130 bzw. von dessen Längsachse L.

Ein der Figur 1 zugeordneter Teillängsschnitt A-A zeigt die Anordnung der Probenträger 200-n im Inneren des Aufnahmeraumes 112 genauer. Konkret ist es zu erkennen, dass die hier beispielhaft fünf gezeigten Probenträger im Inneren des Aufnahmeraumes 112 gestapelt übereinander angeordnet sind. Der Aufnahmeraum 112 bzw. der Stapel von Probenträgern 200-n wird nach oben bzw. zu dem hinteren Ende des Röhrchens hin durch den Stößelkopf 132 des Stößels 130 begrenzt. Diese Begrenzung ist allerdings nicht fix, sondern in Abhängigkeit der Anzahl der aufeinander gestapelten Probenträger variabel.

Das hintere Ende des Röhrchens 110, wo die besagte Durchgangsöffnung angeordnet ist, kann dort als eine radial ausladende Schulter ausgebildet sein zur Erhöhung des Bedienkomforts für eine Bedienperson. Konkret kann die Schulter 118 als Gegenhalt für einen Zeigefinger dienen, wenn das Röhrchen von einer Hand umgriffen wird und mit dem Daumen derselben Hand ein Druck bzw. eine Kraft F auf den Stößelgriff 134 ausgeübt wird.

Die Durchgangsöffnung ist z. B. als ein an die Kontur der Flügel angepasster Schlitz ausgebildet, welcher jeweils einen Flügel nur in einer vorbestimmten Umfangs-Winkellage passieren lässt. Die Durchgangsöffnung kann auch ausgebildet sein, die Flügel in unterschiedlichen Umfangswinkellagen passieren zu lassen; dann ist es jedoch vorteilhaft, wenn diese Passier-Winkellagen an der Durchgangsöffnung nicht identisch sind mit dem Umfangswinkel α zwischen zwei benachbarten Flügeln 140-n, weil die Durchgangsöffnung 114 ansonsten nicht als Anschlag für einen nacheilenden Flügel dienen kann und bei Krafteinwirkung gleich zwei oder noch mehr Probenträger ausgeworfen würden, was nicht gewollt ist.

Figur 2 zeigt die Vorrichtung 100 wie zuvor in Figur 1, jetzt allerdings mit entfernter Kappe 150. Das Entfernen der Kappe erfolgt insbesondere zum Befüllen des Aufnahmeraumes mit den Probenträgern und zum Befüllen der Probenträger mit einer Flüssigkeit, z. B. Blut.

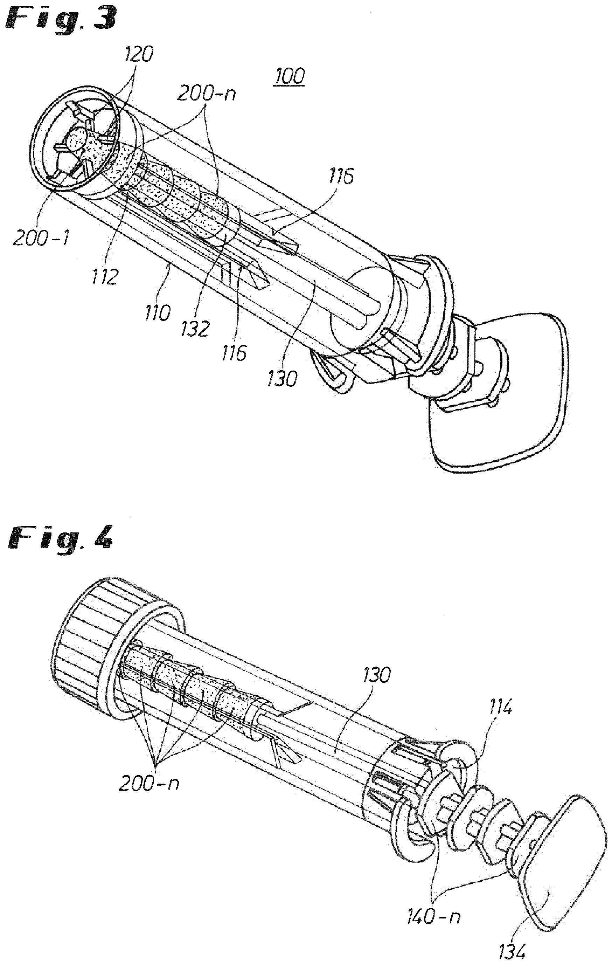

Figur 3 zeigt wiederum eine andere perspektivische Ansicht der erfindungsgemäßen Vorrichtung 100, wobei diese Ansicht vorteilhafterweise einen Einblick in das vordere offene Ende des Röhrchens 110 gewährt. Das Röhrchen 110 ist mit Probenträgern 200-n befüllt, der Aufnahmeraum 112 für die Probenträger ist zu seinem hinteren Ende hin durch den Stößelkopf 132 und an seinem vorderen Ende durch elastische Haltemittel 120 begrenzt. Das Haltemittel 120 kann beispielsweise in Form einer elastischen Lochscheibe oder in Form von mindestens einem elastischen oder elastisch gelagerten Steg ausgebildet sein, welcher sich in radialer Richtung in das Innere des Röhrchens 110 erstreckt. In Figur 3 sind über den Umfang verteilt beispielhaft 4 derartige Haltemittel erkennbar. Das mindestens eine Haltemittel dient zum Rückhalten bzw. Sichern des jeweils vordersten Probenträgers 200-1.

Weiterhin zeigt das Röhrchen 110 gemäß Figur 3 zumindest im Bereich seines Aufnahmeraumes 112 Längsrippen 116, welche sich in Längsrichtung des Röhrchens erstrecken und radial in das Innere des Röhrchens 110 hineinragen zur radialen Begrenzung des Aufnahmeraumes 110. Insbesondere durch die Längsrippen, verteilt in Umfangsrichtung wird der Aufnahmeraum 112 in radialer Richtung so begrenzt, dass sich die Stapellage der Probenträger 200-n entlang der Längsachse L des Röhrchen 110 und vorzugsweise konzentrisch im Inneren des Röhrchens erstreckt und dort durch die Längsrippen 116 gestützt wird. Die Längsrippen sind aus spritzgusstechnischen Gründen vorzugsweise einstückig mit dem Röhrchen 110 und als von der Wand des Röhrchens in das Innere des Röhrchens vorspringende Hohlkörper ausgebildet.

In den Figuren 3 bis 6 sind die Probenträger mit der Flüssigkeit gefüllt, dargestellt durch die Punktierung der Probenträger.

Die erfindungsgemäße Vorrichtung 100 wird wie folgt gehandhabt:
In einem ersten Schritt wird die Kappe 150 an dem vorderen Ende des zunächst noch leeren Röhrchens 110 entfernt. Über das vordere Ende wird dann in einem zweiten Schritt der Aufnahmeraum 112 sequentiell mit einzelnen der Probeträger 200-1 befüllt. Am hinteren Ende des Aufnahmeraumes 112, gestützt durch den Kopf 132 des Stößels und in radialer Richtung gestützt durch die Längsrippen 116, ordnen sich die eingefüllten Probeträger 200-n dann automatisch in einer Stapellage an. An dem vorderen Ende des Aufnahmeraumes wird der erste bzw. vorderste Probenträger 200-1 durch das mindestens eine vorgesehene elastische Haltemittel 120 in dem Aufnahmeraum zurückgehalten, auch dann, wenn die Vorrichtung 100 senkrecht steht und das vordere Ende unten ist.

In einem dritten Schritt wird dann die Flüssigkeit, beispielsweise Blut, durch das vordere offene Ende des Röhrchens zunächst auf den vordersten Probenträger 200-1 aufgebracht. Zunächst saugt sich dann nur der erste Probenträger 200-1 mit der Flüssigkeit voll. Nach dessen Sättigung und der Applizierung von noch mehr Flüssigkeit wandert die Flüssigkeit aufgrund von Kapillarwirkung von Probenträger zu Probenträger, bis schließlich auch der hinterste Probenträger 200-N mit der Flüssigkeit vollgesaugt bzw. gefüllt ist. Sofern der letzte (im Stapel oberste) Probenträger als Reservoir / Puffer für eine überschüssige (nicht bestimmte) Blutmenge benutzt wurde, kann dieser Probenträger im Röhrchen verbleiben und zusammen mit dem Röhrchen entsorgt werden.

In einem vierten Schritt wird dann typischerweise das vordere Ende des Röhrchens 110 mit dem Verschluss 150 verschlossen, siehe Fig. 4. Die Flüssigkeit in den Probenträgen 210 kann dann trocknen und das Röhrchen kann mit den Probenträgern transportiert werden.

Zum Befüllen des Röhrchens mit den Probenträgern ist der Stößel 130 zumindest teilweise, vorzugsweise jedoch weitestgehend aus dem Röhrchen herausgezogen. Je weiter der Stößel herausgezogen ist, desto größer ist der zur Befüllung mit den Probenträgern verfügbare Aufnahmeraum 112.

Jeder der Probenträger 200-n ist ausgebildet, eine jeweils genaue vorbestimmte Menge der Flüssigkeit aufnehmen zu können. Die Befüllung der Vorrichtung 100 mit den Probenträgern und der Flüssigkeit dient dazu, später jeweils genau einen Probenträger mit der in ihm dann enthaltenen Flüssigkeitsmenge separieren zu können; insbesondere in Aufnahmeräume einer Testplatte 300 applizieren zu können.

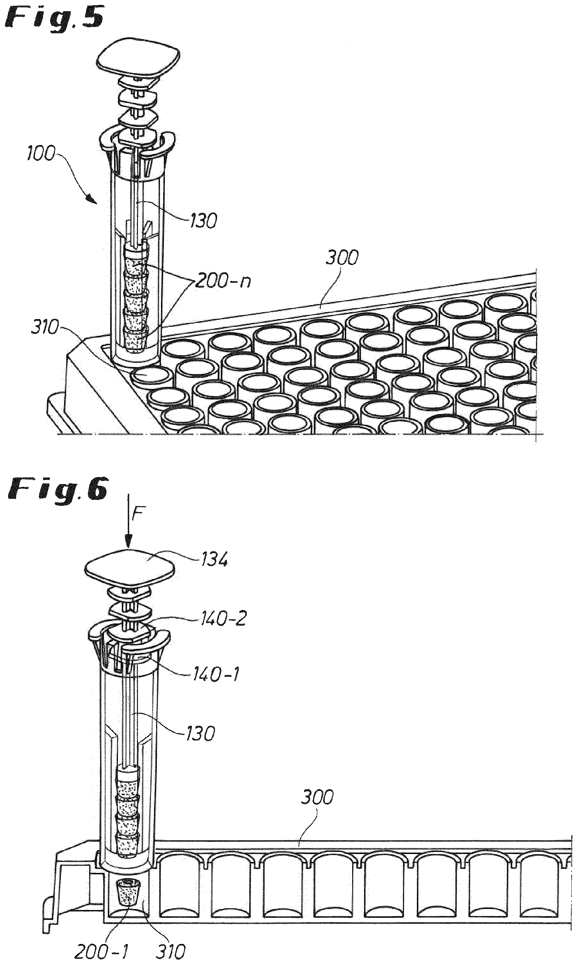

Zu diesem Zweck wird die erfindungsgemäße Vorrichtung mit den enthaltenen befüllten Probenträgern 200-n nach Entfernen der Kappe 150 mit ihrem vorderen offenen Ende auf jeweils einen zu befüllenden Aufnahmeraum 310 aufgesetzt, wie dies in Figur 5 gezeigt ist.

Figur 6 zeigt die Abgabe des ersten bzw. vordersten Probenträgers 200-1 in den Aufnahmeraum 310 der Testplatte 300. Zu diesem Zweck wird typischerweise mit der Hand bzw. dem Daumen einer Bedienperson eine Kraft F von oben auf den Stößelgriff 134 ausgeübt. Dabei ist es wichtig, dass die Winkellage des voreilenden, vor der Durchgangsöffnung 114 anstehenden Flügels der Umfangswinkellage der Durchgangsöffnung 114 entspricht. Nur dann bewirkt die besagte Kraft F, dass der voreilende Flügel 140-1 die Durchgangsöffnung 114 passiert und die Kraft F über den Stößel 130 und den Stößelkopf 132 zunächst auf den hintersten Probenträger 200-N übertragen wird. Aufgrund der gestapelten und durch die Längsrippen 116 radial gestützten Anordnung der Probenträger überträgt sich die Kraft dann von dem hintersten Probenträger 200-N auf den vordersten Probenträger 200-1 und auf das Haltemittel 120. Ist die Kraft F groß genug, so wird ein Rückhaltewiderstand des Haltemittels 120 überwunden und der vorderste Probenträger 200-1 wird dann aus dem Aufnahmeraum 112 des Röhrchens in die Öffnung 310 der Testplatte 300 herausgedrückt.

Das sofortige nachfolgende Herausdrücken des zweiten Probenträgers 200-2 aus dem Aufnahmeraum wird dadurch verhindert, dass die Weglänge, um welche der Stößel 130 durch die Krafteinwirkung in das Röhrchen hineingedrückt wird, auf den Auswurfhub, d. h. den Abstand zwischen zwei benachbarten Flügeln an dem Stößel begrenzt ist. Diese Begrenzung ist bei der erfindungsgemäßen Vorrichtung 100 konstruktiv durch den Versatz von zwei benachbarten Flügeln in Umfangsrichtung realisiert. Der voreilende Flügel 140-1 kann, wie gesagt, die Durchlassöffnung 114 deshalb passieren, weil seine Winkellage mit der Durchlasswinkellage der Durchlassöffnung korrespondiert. Der nachfolgende bzw. nacheilende Flügel 140-2 hat jedoch zunächst, ohne dass der Stößel in Umfangsrichtung gedreht wurde, eine andere, nicht mit der Durchlass-Winkellage der Durchlassöffnung 114 korrespondierende eigene Winkellage. Weil diese Winkellage zunächst nicht mit der Durchlass-Winkellage der Durchlassöffnung 114 korrespondiert, schlägt der nacheilende Flügel 140-2 zunächst nach dem Passieren des voreilenden Flügels unter der Krafteinwirkung an der Durchlassöffnung an. Insofern ist die Weglänge, um die sich der Stößel bei Krafteinwirkung bewegt, bzw. der Auswurfhub auf den Abstand zwischen dem voreilenden und dem nacheilenden Flügel begrenzt. Dieser Auswurfhub entspricht typischerweise dem Durchmesser bzw. der Länge bzw. der Höhe eines der Probenträger 200-n. Soll dann nachfolgend der zweite Probenträger typischerweise in einen anderen Aufnahmeraum 310 der Testplatte 300 ausgeworfen werden, so muss zuvor der Stößel 130 so gedreht werden, dass der zuvor nacheilende, jetzt voreilende Flügel in seiner Winkellage mit der Passier-Winkellage der Durchgangsöffnung korrespondiert.

### Bezugszeichenliste

- 100: Vorrichtung
- 110: Röhrchen
- 112: Aufnahmeraum des Röhrchens
- 114: Durchgangsöffnung
- 116: Längsrippen
- 118: Schulter
- 120: Haltemittel
- 130: Stößel
- 132: Stößelkopf
- 134: Stößelgriff
- 140-1: voreilender Flügel
- 140-2: nacheilender Flügel
- 140-n: Flügel
- 150: Verschluss
- 200-n: Probenträger
- 300: Testplatte
- 310: Aufnahmerau der Testplatte

- a: Abstand
- L: Längsrichtung
- α: Umfangswinkel
- U: Umfangsrichtung
- F: Kraft

## Patentansprüche

1. Vorrichtung (100) zum Bereitstellen von Probenträgern (200-n), welche jeweils ausgebildet sind, eine vorbestimmte Menge einer Flüssigkeit, beispielsweise Blut, aufzunehmen, aufweisend:
ein Röhrchen (110) mit einem Aufnahmeraum (112) in seinem Innern zur Aufnahme einer Mehrzahl der Probenträger, gestapelt in Längsrichtung (L) des Röhrchens (110);
mindestens ein an einem vorderen Ende des Röhrchens angeordnetes Haltemittel (120) zum einseitigen Begrenzen des Aufnahmeraumes; und
einen an dem hinteren Ende des Röhrchens in Längsrichtung (L) des Röhrchens verschiebbar gelagerten Stößel (130), welcher mit seinem einen Ende in Form eines Stößelkopfes (132) in das Röhrchen (110) eintaucht zum Begrenzen des Aufnahmeraumes gegenüberliegend zu dem Haltemittel (120), und welcher mit seinem anderen Ende in Form eines Stößelgriffes (134) aus dem Röhrchen herausragt,
**dadurch gekennzeichnet,**
**dass** das Haltemittel (120) elastisch ausgebildet ist einerseits zum Halten der Probenträger (200-n) in dem Aufnahmeraum (112) und andererseits zum kontrollierten vereinzelten Freigeben der Probenträger (200-n) aus Aufnahmeraum (112) unter Krafteinwirkung durch den Stößel (130);
**dass** an dem Stößel (130) zumindest im Bereich seines Stößelgriffes ein Mehrzahl von Flügeln (140-n) in Längsrichtung (L) des Stößels um einen Abstand a beabstandet zueinander angeordnet ist, wobei benachbarte der Flügel in Umfangsrichtung (U) des Stößels (130) jeweils um einen vorbestimmten Umfangswinkel α zueinander versetzt sind; und
**dass** an dem hinteren Ende des Röhrchens (110) eine Durchgangsöffnung (114) vorgesehen ist, welche ausgebildet ist, die Flügel (140-n) des Stößels (130) nur in mindestens einer vorbestimmten Umfangswinkellage passieren zu lassen.

2. Vorrichtung (100) nach Anspruch 1,
**dadurch gekennzeichnet,**
**dass** sich die Flügel (140-n) jeweils radial von dem Stößel (130) erstrecken.

3. Vorrichtung (100) nach einem der vorangegangenen Ansprüche,
**dadurch gekennzeichnet,**
**dass** der Umfangswinkel a, um welchen jeweils zwei benachbarte der Flügel versetzt zueinander auf dem Stößel angeordnet sind, beispielsweise α = 90° beträgt.

4. Vorrichtung (100) nach einem der vorangegangenen Ansprüche,
**dadurch gekennzeichnet,**
**dass** der Abstand a zwischen jeweils zwei benachbarten Flügeln zumindest näherungsweise dem Durchmesser oder der Längserstreckung eines der Probenträger (200-n) entspricht.

5. Vorrichtung (100) nach einem der vorangegangenen Ansprüche,
**dadurch gekennzeichnet,**
**dass** das Röhrchen (110) zumindest im Bereich des Aufnahmeraumes (112) über seinen Umfang verteilt Längsrippen (116) aufweist, welche sich in Längsrichtung des Röhrchens erstrecken und radial in das Innere des Röhrchens hineinragen zur radialen Begrenzung des Aufnahmeraumes.

6. Vorrichtung (100) nach Anspruch 5,
**dadurch gekennzeichnet,**
**dass** die Längsrippen (116) einstückig mit dem Röhrchen (110) und als von der Wand des Röhrchens in das Innere des Röhrchens vorspringende Hohlkörper ausgebildet sind.

7. Vorrichtung (100) nach einem der vorangegangenen Ansprüche,
**gekennzeichnet durch**
einen Verschluss (150), beispielsweise in Form einer Kappe oder eines Stopfens zum Verschließen des vorderen Endes des Röhrchens (110).

8. Vorrichtung (100) nach einem der vorangegangenen Ansprüche,
**dadurch gekennzeichnet,**
**dass** das Röhrchen (110) aus einem transparenten Material, beispielsweise Glas oder Kunststoff gefertigt ist.

9. Vorrichtung (100) nach einem der vorangegangenen Ansprüche,
**dadurch gekennzeichnet,**
**dass** das Haltemittel (120) in Form einer elastischen Lochscheibe oder in Form von mindestens einem elastischen Steg oder einem elastisch gelagerten Steg ausgebildet ist, welche/welcher sich in radialer Richtung in das Innere des Röhrchens (110) hinein erstreckt.

## Claims

1. A device (100) for providing sample carriers (200-n) which are each constructed to receive a predetermined quantity of a liquid, for example blood, comprising:
a tube (110) with a compartment (112) in its interior for receiving a plurality of sample carriers stacked in the longitudinal direction (L) of the tube (110);
at least one retaining means (120) which is disposed at a front end of the tube in order to delimit one end of the compartment; and
a plunger (130) which is displaceably mounted at the rear end of the tube in the longitudinal direction (L) of the tube, with one end in the form of a plunger head (132) being inserted into the tube (110) in order to delimit the compartment opposite the retaining means (120), and with the other end, in the form of a plunger grip (134), protruding out of the tube;
**characterized in that**
the retaining means (120) is resilient in construction, on the one hand in order to retain the sample carriers (200-n) in the compartment (112), and on the other hand in order to provide controlled individual release of the sample carriers (200-n) from the compartment (112) under the effect of force applied via the plunger (130);
**in that** a plurality of fins (140-n) are disposed on the plunger (130) at least in the region of the plunger grip thereof, spaced from one another in the longitudinal direction (L) of the plunger by a distance a, wherein adjacent fins in the circumferential direction (U) of the plunger (130) are respectively offset relative to one another by a predetermined circumferential angle α; and
a through opening (114) is provided at the rear end of the tube (110) which is constructed to allow the fins (140-n) of the plunger (130) to pass through only in at least one predetermined circumferential angular position.

2. The device (100) as claimed in claim 1,
**characterized in that**
the fins (140-n) respectively extend radially from the plunger (130).

3. The device (100) as claimed in one of the preceding claims,
**characterized in that**
the circumferential angle α by which respectively adjacent fins are offset to one another on the plunger is α = 90°, for example.

4. The device (100) as claimed in any one of the preceding claims,
**characterized in that**
the spacing a between two respectively adjacent fins at least approximately corresponds to the diameter or the longitudinal extent of one of the sample carriers (200-n) .

5. The device (100) as claimed in any one of the preceding claims,
**characterized in that**
at least in the region of the compartment (112), the tube (110) has longitudinal ribs (116) which are distributed over its circumference, which extend in the longitudinal direction of the tube and which protrude radially into the interior of the tube in order to radially delimit the compartment.

6. The device (100) as claimed in claim 5,
**characterized in that**
the longitudinal ribs (116) are formed as one piece with the tube (110) and are constructed as hollow bodies protruding from the wall of the tube into the interior of the tube.

7. The device (100) as claimed in one of the preceding claims,
**characterized by**
a closure (150), for example in the form of a cap or a plug, in order to close the front end of the tube (110) .

8. The device (100) as claimed in one of the preceding claims,
**characterized in that**
the tube (110) is produced from a transparent material, for example glass or plastic.

9. The device (100) as claimed in any one of the preceding claims,
**characterized in that**
the retaining means (120) is constructed in the form of a resilient apertured disc or in the form of at least one resilient web or a resiliently mounted web, which extends into the interior of the tube (110) in the radial direction.

## Revendications

1. Dispositif (100) pour fournir des supports d'échantillon (200-n), lesquels sont conçus respectivement pour recevoir une quantité prédéterminée d'un liquide, par exemple du sang, présentant :
un petit tube (110) avec un espace de réception (112) dans son intérieur pour recevoir une pluralité des supports d'échantillon, empilés dans le sens longitudinal (L) du petit tube (110) ;
au moins un moyen de retenue (120) disposé à une extrémité avant du petit tube pour délimiter l'espace de réception sur un côté ; et
un poussoir (130) disposé sur l'extrémité arrière du petit tube en étant mobile dans le sens longitudinal (L) du petit tube, lequel pénètre dans le petit tube (110) par une extrémité sous forme d'une tête de poussoir (132) pour délimiter l'espace de réception face au moyen de retenue (120) et lequel sort du petit tube par son autre extrémité sous forme d'une préhension de poussoir (134),
**caractérisé en ce que**
le moyen de retenue (120) est élastique d'une part pour retenir les supports d'échantillon (200-n) dans l'espace de réception (112) et d'autre part pour libérer de façon individualisée et contrôlée les supports d'échantillon (200-n) hors de l'espace de réception (112) par application de force par le poussoir (130) ;
que sur le poussoir (130), au moins au niveau de sa préhension de poussoir, une pluralité d'ailettes (140-n) est disposée dans le sens longitudinal (L) du poussoir à une distance a l'une de l'autre, dans lequel des ailettes voisines sont décalées respectivement l'une par rapport à l'autre d'un angle α périphérique prédéterminé, dans le sens périphérique (U) du poussoir (130) ; et
que sur l'extrémité arrière du petit tube (110), une ouverture de passage (114) est prévue qui est conçue pour laisser passer les ailettes (140-n) du poussoir (130) uniquement dans au moins une position angulaire périphérique prédéterminée.

2. Dispositif (100) selon la revendication 1,
**caractérisé en ce que**
les ailettes (140-n) s'étendent respectivement radialement à partir du poussoir (130).

3. Dispositif (100) selon l'une des revendications précédentes,
**caractérisé en ce que**
l'angle α périphérique autour duquel respectivement deux ailettes voisines sont disposées sur le poussoir en étant décalées l'une par rapport à l'autre, fait par exemple α = 90°.

4. Dispositif (100) selon l'une des revendications précédentes,
**caractérisé en ce que**
l'écart a entre respectivement deux ailettes voisines correspondant au moins approximativement au diamètre ou à l'allongement longitudinal d'un des supports d'échantillon (200-n).

5. Dispositif (100) selon l'une des revendications précédentes,
**caractérisé en ce que**
le petit tube (110) présente des nervures longitudinales (116) réparties sur son pourtour, au moins au niveau de l'espace de réception (112), lesquelles s'étendent dans le sens longitudinal du petit tube et pénètrent radialement dans l'intérieur du petit tube pour délimiter radialement l'espace de réception.

6. Dispositif (100) selon la revendication 5,
**caractérisé en ce que**
les nervures longitudinales (116) sont formées d'une seule pièce avec le petit tube (110) et en tant que corps creux faisant saillie de la paroi du petit tube jusque dans l'intérieur du petit tube.

7. Dispositif (100) selon l'une des revendications précédentes,
**caractérisé par**
une fermeture (150), par exemple sous forme d'un capuchon ou d'un bouchon pour fermer l'extrémité avant du petit tube (110).

8. Dispositif (100) selon l'une des revendications précédentes,
**caractérisé en ce que**
le petit tube (110) est fabriqué dans un matériau transparent, par exemple du verre ou du plastique.

9. Dispositif (100) selon l'une des revendications précédentes,
**caractérisé en ce que**
le moyen de retenue (120) est conçu sous forme d'un disque percé élastique ou sous forme d'au moins une branche élastique ou d'une branche placée de manière élastique, lequel/laquelle s'étend dans le sens radial jusque dans l'intérieur du petit tube (110).
